# EUROPÄISCHE PATENTSCHRIFT

(11) **EP 2 964 232 B1**
(45) Veröffentlichungstag und Bekanntmachung des Hinweises auf die Patenterteilung: **02.10.2019**
(21) Anmeldenummer: 14716247.3
(22) Anmeldetag: 06.03.2014
(51) Int. Cl.: A61K 31/5415, A61P 31/22

(54) **PIROXICAM ZUR ANWENDUNG BEI DER PROPHYLAKTISCHEN UND THERAPEUTISCHEN BEHANDLUNG VON CMV-INFEKTIONEN**
PIROXICAM FOR USE IN THE PROPHYLACTIC AND THERAPEUTIC TREATMENT OF CMV INFECTIONS
PIROXICAM POUR UTILISATION LORS DU TRAITEMENT PROPHYLACTIQUE ET THÉRAPEUTIQUE D'INFECTIONS À CMV

(30) Priorität: 06.03.2013 DE 102013003756
(43) Veröffentlichungstag der Anmeldung: 13.01.2016
(73) Patentinhaber: Medicocensus GmbH, 30625 Hannover (DE)
(72) Erfinder: MEYER ZU SPELBRINK, Hans-Otto, 30169 Hannover (DE); ALBRECHT, Uwe, 31303 Burgdorf (DE)
(74) Vertreter: Thiel, Christian
(86) Internationale Anmeldenummer: PCT/EP2014/054353
(87) Internationale Veröffentlichungsnummer: WO 2014/135638

(56) Entgegenhaltungen:
- WO-A1-2011/124366
- J. SCHROER ET AL: "Inhibition of cyclooxygenase activity blocks cell-to-cell spread of human cytomegalovirus", PROCEEDINGS OF THE NATIONAL ACADEMY OF SCIENCES, Bd. 105, Nr. 49, 9. Dezember 2008 (2008-12-09), Seiten 19468-19473, XP055124462, ISSN: 0027-8424, DOI: 10.1073/pnas.0810740105
- ZHU H ET AL: "INHIBITION OF CYCLOOXYGENASE 2 BLOCKS HUMAN CYTOMEGALOVIRUS REPLICATION", PROCEEDINGS OF THE NATIONAL ACADEMY OF SCIENCES, NATIONAL ACADEMY OF SCIENCES, US, Bd. 99, Nr. 6, 19. März 2002 (2002-03-19), Seiten 3932-3937, XP000962813, ISSN: 0027-8424, DOI: 10.1073/PNAS.052713799

## Beschreibung

Die Erfindung betrifft eine Zusammensetzung zur Verwendung bei der prophylaktischen und therapeutischen Behandlung von Virusinfektionen mit dem Cytomegalievirus.

Die Behandlung von Virusinfektionen in Mensch und Tier stellt nach wie vor eine Herausforderung dar, da die Zahl der Wirkstoffe begrenzt ist. Dies gilt auch für die Viren der Herpesfamilie.

Die Familie der Herpesviren (Herpesviridae) umfasst eine große Anzahl von Viren mit doppelsträngiger DNA. Weit verbreitet sind die Herpes simplex-Viren vom Typ HSV1 und HSV2 sowie das Variceller-Zoster-Virus VZV. Alle verursachen schmerzhafte Infektionen, die sich in oberflächlichen Entzündungen äußern. Herpesviren überdauern lange Zeit im menschlichen Körper, so dass es immer wieder zu Ausbrüchen der Krankheit mit zum Teil gravierenden Krankheitserscheinungen kommt.

Zu den Herpesviren gehört das Cytomegalievirus, CMV, auch als HHV5 bezeichnet. Wie auch andere Viren der Herpesfamilie verbleibt es nach der Erstinfektion lebenslang im menschlichen Körper und kann - insbesondere unter Stressbedingungen - jederzeit wieder aktiv werden.

Die Erstinfektion mit dem Cytomegalievirus bleibt meist unbemerkt; vielfach verläuft sie symptomlos oder mit nur leichtem Fieber und Müdigkeitserscheinungen, die allgemein unter "grippaler Infekt" verbucht werden. Bis zu 60% der Bevölkerung sind Träger des Cytomegalievirus. Es bleibt lebenslang im lymphatischen Gewebe erhalten.

Eine "Zweitinfektion" wie sie unter Stresserscheinungen auftreten kann, kann dagegen zu schweren Komplikationen führen. Neben grippeähnlichen Erscheinungen mit Fieber, Schwellung der Lymphknoten und Kopf- wie Gliederschmerzen treten Infektionen des Darms (CMV-Colitis), der Leber (CMV-Hepatitis) und der Lunge (CMV-Pneumonie) auf.

Gravierend sind CMV-Infektionen während der Schwangerschaft, die häufig zu Fehlgeburten und schweren Fehlbildungen beim Kind führen. Auch während der Stillzeit kann das Virus übertragen werden.

Weiterhin treten CMV-Infektionen gehäuft nach Transplantationen und bei Therapien mit Cytostatika oder Immunsuppressiva auf. Bei Nierentransplantationen erleiden mehr als 30% aller Patienten, die eine neue Niere erhalten haben, eine solche Infektion.

Gegen Herpesviren wurde eine Reihe von Mitteln entwickelt, die im Wesentlichen alle nur geeignet sind, die Symptome zu lindern, aber nur einen geringen Einfluss auf den Verlauf der Krankheit selbst nehmen. Zumeist kommt es nur zu einer Verkürzung der Krankheitsdauer. Ein viel eingesetzter Wirkstoff ist Aciclovir. Weitere, neu entwickelte Wirkstoffe sind Ganciclovir und Foscamet. Alle diese Wirkstoffe sind Virostatika.

Grundsätzlich besteht ein Bedarf an Mitteln, mit denen einer CMV-Infektion wirksam begegnet werden kann. Dabei wäre ein Wirkstoff von besonderem Interesse, der nicht nur virostatisch, sondern darüber hinaus auch virozid wirkt.

Aus der EP 1 457 202 A2 ist die Verwendung von sog. nicht-steroiden entzündungshemmenden Mitteln (NSAIDs) zur Behandlung von Herpesinfektionen beschrieben. Obwohl in der Anmeldung eine große Anzahl von NSAIDs genannt wird, wird eine Wirksamkeit nur für zwei Vertreter dieser Gruppe, Diclofenac und Ketorolac beschrieben und nur für Diclofenac anhand belastbarer Daten belegt. Demnach ist Diclofenac in topischer Anwendung geeignet, den Krankheitsverlauf zu mildern; im Schnitt wird eine Abheilung der Läsionen nach fünf Tagen erreicht. Dies ist zwar eine Verkürzung der normalen Infektionsdauer, die bis zu 10 Tage dauert, jedoch für den Patienten immer noch unbefriedigend. Eine Behandlung von Cytomegalieinfektionen wird nicht beschrieben.

Schröer et al (PROCEEDINGS OF THE NATIONAL ACADEMY OF SCIENCES, 2008, vol. 105, no. 49, p. 19468-19473) und Zhu et al (PROCEEDINGS OF THE NATIONAL ACADEMY OF SCIENCES, 2002, vol. 99, no. 6, p. 3932-3937) beschreiben experimentelle Versuche mit COX-Inhibitoren, welche die Hemmung von humanem CMV dokumentieren.

Aus der WO 2011/124366 A1 ist die Verwendung von Piroxicam zur Behandlung von HSV1-, HSV2- und VSV-Infektionen bekannt. Alle diese Viren der Herpesfamilie gehören zur Unterfamilie der Alphaherpesvirinae. Das CMV gehört zu einer anderen Unterfamilie, den Betaherpesvirinae. Eine Wirksamkeit von Piroxicam bei CMV-Infektionen ist weder beschrieben noch nahegelegt. Aus der Wirksamkeit bei HSV-und VSV-Infektionen kann die Einsetzbarkeit bei CMV-Infektionen nicht hergeleitet werden.

Es wurde jetzt überraschend gefunden, dass Piroxicam geeignet ist, CMV-Infektionen prophylaktisch und therapeutisch zu behandeln. Piroxicam, 4-Hydroxy-2-methyl-N-pyridyl-2-yl-2H-1,2-benzothiazin-3-carboxamid-1,1-dioxid, ist ein COX-Hemmer und wird als stark wirkendes Antirheumatikum eingesetzt.

Entsprechend betrifft die Erfindung eine Zusammensetzung der eingangs genannten Art, das Piroxicam in einer geeigneten Trägersubstanz enthält.

Die Erfindung betrifft ferner Piroxicam zur Verwendung bei der Herstellung eines Arzneimittels zur Behandlung von CMV-Infektionen bzw. zur Vorbeugung derselben.

Die erfindungsgemäße Zusammensetzung enthält Piroxicam vorzugsweise in einer Menge von 0,1 bis 10 Gew.-%, insbesondere von 0,1 bis 5 Gew.-% und besonders bevorzugt in einer Menge von 1 bis 5 Gew.-%. Es kann oral und parenteral verabreicht werden.

Für die orale Verabreichung liegt die Zusammensetzung in üblicher Weise in einer bei Tabletten üblichen Trägersubstanz vor. Eine Verabreichung in Pulverform, beispielsweise durch Einrühren in eine Flüssigkeit, ist ebenfalls möglich. Daneben sind übliche Injektions- und Infusionslösungen als Verabreichungsform geeignet.

Die Verabreichung erfolgt im Allgemeinen analog zur Verabreichung von Piroxicam bei der Behandlung von Rheumaerkrankungen. Auch die Dosierung entspricht der Dosierung bei Rheumaerkrankungen.

### Testbericht

In einem Screening-Test wurde eine Injektionslösung "Piroxicam parenteral 20mg" auf ihre virusinaktivierenden Eigenschaften gegenüber dem Cytomegalievirus untersucht. Die Wirksamkeit wurde ohne organische Belastung geprüft. Das Prüfprodukt wurde konzentriert (20mg/ml) geprüft. Es wurden folgende Einwirkzeiten getestet: 30s, 60s und 300s.

Die Prüftemperatur betrug 20,0 ± 0,5°C. Als Virusstamm wurde der CMV-Stamm AD169 (ATCC, VR-538) eingesetzt, als Zelllinie embryonale Lungenfibroblasten. Zur Herstellung der Virussuspension wurden konfluente Monolayer von embryonalen Lungenfibroblasten eingesetzt. Die Lagerung der aliquotierten Arbeitsvirussuspension erfolgte bei -70°C.

Als Prüfgemisch wurden 100µl Injektionslösung mit 49,9ml Wasser verdünnt.

Das Prüfgemisch bestand aus 0,1ml Virussuspension, 0,1ml destilliertem Wasser und 0,8ml unverdünnter Injektionslösung (20mg/ml).

Unmittelbar nach der Einwirkzeit wurde die virozide Wirkung durch das neunfache Volumen eisgekühlten Zellkulkturerhaltungsmediums unterdrückt. Anschließend wurde sofort eine Titration in 10er-Schritten mit eisgekühltem MEM mit FKS angesetzt und die Verdünnungsreihe umgehend auf eine konfluent gewachsene Zellkultur übertragen.

Der Nachweis der mit Cytomegalievirus infizierten Zellen erfolgte mittel Immuno-Peroxidase-Färbung nach Eggers et al., 1998 (J. Med. Viro. 56, 351-358). Der Virustiter wurde im Quantalversuch (Endpunkttitration) bestimmt. Zunächst wurde eine Verdünnungsreihe in 10er-Schritten der zu untersuchenden Virussuspensionen hergestellt (20µl Virussuspension + 180µl MEM). Je sechs Vertiefungen einer 96-well-Platte konfluent gewachsener ELU-Zellen wurden mit 100µl der Verdünnungsreihen inokuliert und die Platte bei 1200 x g für 30 Minuten zentrifugiert und danach das Medium gewechselt. Die beimpften Zellkulturen wurden für 72 Stunden bei 37°C mit 5% CO₂ in der feuchten Kammer inkubiert.

Nach der Bebrütung wurde das Medium von den Zellkulturplatten abgesaugt und die Zellen mit eiskaltem Aceton-Methanol-Gemisch (40:60) fixiert. Anschließend wurden die Platten mit 100µl Blockingpuffer für 30min bei 37°C und 5% CO₂ überschichtet. Es erfolgte die Zugabe eines CMV-spezifischen monoklonalen Antikörpergemischs (Monoclonal-Mouse-Anti-Cytomegalievirus-Clones CCH2+DDG, Fa. Dako/Deutschland) in einer Verdünnung von 1:200 in Blocking-Puffer (1% BSA in PBS). Nach 30-minütiger Inkubation wurde die Antikörpermischung verworfen und dreimal mit PBS gewaschen. Anschließend erfolge eine 30-minütige Inkubation mit einem Anti-Mouse-Horseradish-Peroxidase-markierten Konjugat (Fa. Dako/Deutschland) in der Verdünnung 1:500. Das im nachfolgenden Schritt zugefügte Substrat (AEC) reagierte mit dem HRP-markierten Konjugat, wobei es zu einer sichtbaren Farbumwandlung (Rotfärbung) kam. Virusinfizierte Zellen ließen sich mikroskopisch anhand spezifischer Färbung der Zellen erkennen.

Es wurde die Virusverdünnung ermittelt, bei der 50% der beimpften Kulturen eines Verdünnungssatzes infiziert waren (TCID₅₀/ml). Die Berechnung der TCID₅₀/ml erfolgte nach dem Spearman-Kärber-Verfahren (Br. J. Psychol. 2:227-42, 1908; Arch. exp. Path. Pharmak. 162:480-7, 1931).

Die Beurteilung der Reduktion erfolgte durch die Berechnung des Titerabfalls gegenüber der jeweils parallel mitgeführten Viruskontrolle.

| **Konzentration des Prüfprodukts** | **Virustiter der Kontrolltitration (log₁₀ TCID₅₀/ml) einschließlich 95% Konfidenzintervall** | **Virustiter der "Restvirus"-Titration (log₁₀ TCID₅₀/ml) einschließlich 95% Konfidenzintervall** | | | **Reduktionsfaktor einschließlich 95% Konfidenzintervall** | | |
|---|---|---|---|---|---|---|---|
| | | 30s | 60s | 300s | 30s | 60s | 300s |
| konzentriert (20mg/ml) | 6,33 +/-0,54 | ≤ 1,50 | ≤ 1,50 | ≤ 1,50 | ≤ 4,83 | ≤ 4,83 | ≤ 4,83 |
| | | +/- 0,00 | +/- 0,00 | +/- 0,00 | +/- 0,54 | +/- 0,54 | +/- 0,54 |

Für die unverdünnte Injektionslösung (20mg/ml) konnte nach 30s eine Reduktion des Cytomegalievirus um 4,83 log₁₀-Stufen nachgewiesen werden.

## Patentansprüche

1. Zusammensetzung zur Verwendung bei der prophylaktischen und therapeutischen Behandlung von CMV-Infektionen, **dadurch gekennzeichnet, dass** es in einer Trägersubstanz Piroxicam enthält.

2. Zusammensetzung zur Verwendung nach Anspruch 1, **gekennzeichnet durch** ein Gehalt von 0,1 bis 10 Gew.-% Piroxicam.

3. Zusammensetzung zur Verwendung nach Anspruch 2, **gekennzeichnet durch** ein Gehalt an Piroxicam von 1 bis 5 Gew.-%.

4. Zusammensetzung zur Verwendung nach einem der vorstehenden Ansprüche in Form einer Tablette oder Injektionslösung.

5. Zusammensetzung zur Verwendung nach einem der Ansprüche 1 bis 3 zur Infusion.

6. Piroxicam zur Verwendung bei der prophylaktischen und therapeutischen Behandlung von CMV-Infektionen.

## Claims

1. Composition for use in the prophylactic and therapeutic treatment of CMV-infections, **characterized in that** said composition contains piroxicam in a carrier substance.

2. Composition for use according to claim 1, **characterized by** a content of piroxicam ranging between 0.1 and 10 % w/w.

3. Composition for use according to claim 2, **characterized by** a content of piroxicam ranging between 1 and 5 % w/w.

4. Composition for use according to any one of the above claims in the form of a tablet or infusion solution.

5. Composition for use according to any one of the claims 1 to 3 for infusion.

6. Piroxicam for use in the prophylactic and therapeutic treatment of CMV-infections.

## Revendications

1. Composition pour une utilisation dans le traitement prophylactique et thérapeutique d'infections par le CMV (cytomégalovirus), **caractérisée en ce qu'**elle contient du piroxicam dans une substance de support.

2. Composition pour une utilisation selon la revendication 1, **caractérisée par** une teneur en piroxicam de 0,1 à 10% en poids.

3. Composition pour une utilisation selon la revendication 2, **caractérisée par** une teneur en piroxicam de 1 à 5% en poids.

4. Composition pour une utilisation selon l'une quelconque des revendications précédentes, sous forme d'un comprimé ou d'une solution d'injection.

5. Composition pour une utilisation selon l'une quelconque des revendications 1 à 3 destinée à une perfusion.

6. Piroxicam pour une utilisation dans le traitement prophylactique et thérapeutique d'infections par le CMV.
